# EUROPÄISCHE PATENTANMELDUNG

(11) **EP 1 072 891 A2**
(43) Veröffentlichungstag der Anmeldung: **31.01.2001**
(21) Anmeldenummer: 00115083.8
(22) Anmeldetag: 27.07.2000
(51) Int. Cl.: G01N 33/68, G01N 33/566, G01N 33/563, G01N 33/577

(54) **Nachweis einer irreversiblen Schädigung von IgG Antikörpern**

(30) Priorität: 29.07.1999 DE 19935794; 20.09.1999 DE 19944995
(71) Anmelder: GSF-Forschungszentrum für Umwelt und Gesundheit GmbH, 85764 Neuherberg (DE)
(72) Erfinder: Kummer, Udo, Dr., 80538 München (DE)
(74) Vertreter: Reinhard - Skuhra - Weise & Partner

(57) **Zusammenfassung**

Die Erfindung beschreibt ein Verfahren zum Nachweis einer irreversiblen Schädigung von IgG Antikörpern.

## Beschreibung

Die vorliegende Erfindung betrifft ein Verfahren zum Nachweis einer irreversiblen Schädigung von IgG Antikörpern.

Im diagnostischen Bereich und als therapeutische Maßnahme werden monoklonale Antikörper immer häufiger beim Menschen eingesetzt. Ein gravierendes Problem, das im Umgang mit monoklonalen Antikörpern immer wieder auftreten kann, besteht darin, daß gebräuchliche Aufreinigungsprozeduren, wie die EinSchritt-Affinitätschromatographie mit Protein A oder G, das Bindungsverhalten des Antikörpers beeinträchtigen. Thermodynamische Untersuchungen der letzten Jahre konnten nun aufdecken, daß im Verlaufe der Evolution viele Proteine auf geringe Stabilität hin selektiert wurden. Das gilt auch für Immunglobuline und erklärt deren Anfälligkeit gegenüber Denaturierung, z.B. durch Säuren. Die Tatsache, daß der Rückfaltungsprozess zum nativ gefalteten Protein nach Denaturierung in vitro nicht immer vollständig verläuft, kann darüber hinaus erklären, daß gewisse Reinigungsprozeduren zu einem Totalverlust der Bindungsaktivität des monoklonalen Antikörpers führen. Eine unvollständige Rückfaltung des Antikörpers kann aber auch Regionen des Moleküls betreffen, die nicht direkt Einfluß auf das Bindungsverhalten des Antikörpers nehmen. Dieser Zusammenhang erschließt sich zwangsläufig aus der Beobachtung, daß Domänen eines Proteins sich unabhängig voneinander falten können; dies gilt auch für Domänen von Antikörpermolekülen. (Literatur siehe Lehrbücher der Biochemie, z.B. Biochemie von Lubert Stryer). Wenn z.B. der Fc-Teil des Antikörpers von diesen Unregelmäßigkeiten betroffen ist, kann sich das auf seine Fähigkeit auswirken, mit Fc-Rezeptor-tragenden Effektorzellen (Monozyten/Makrophagen, Granulozyten, follikuläre dendritische Zellen, natürliche Killerzellen, Mastzellen), den Exekutoren der angeborenen Immunität, zu interagieren. Dieser Effektorfunktion kommt umsomehr Bedeutung zu, als jüngste Untersuchungen zu dem Schluß kommen, daß IgG Antikörper in vivo ausschließlich über die Interaktion mit FcγRezeptor-tragenden Zellen biologische Wirksamkeit entfalten, die, einmal aktiviert, sich in Phagozytose, Entzündung und Zelllyse manifestiert. (J.V. Ravetch and R.A.Clynes. 1998. Annu Rev Immunol 16:421-432).

Um das Risiko für den Patienten so gering wie möglich zu halten, gibt es seit mehr als 10 Jahren behördliche Auflagen, die besondere Qualitätskriterien für diejenigen Antikörperpräparationen vorschreiben, die für den Gebrauch im Menschen bestimmt sind (Haase M., 1987. Behördliche Anforderungen an die Herstellung und Prüfung von monoklonalen Antikörpern. Pharma Technol 4:32-35). Im Endprodukt soll der gewünschte monoklonale Antikörper zu mehr als 90% angereichert sein, wobei mindestens 95% des Immunglobulins in Form von Monomeren und Dimeren vorliegen. Des weiteren müssen für jeden Antikörper funktionelle Eigenschaften wie Bindungskonstante und komplementabhängige Zytoloyse bekannt und dokumentiert sein.

Eine Überprüfung auf eine irreversible Schädigung von IgG Antikörpern ist bisher kein fester Bestandteil der Qualitätskontrolle, auch nicht bei denjenigen Antikörperpräparationen, die für den Einsatz im Menschen vorgesehen sind (z.B. Panorex, siehe Rote Liste; OKT3).

Es ist eine Aufgabe der vorliegenden Erfindung, ein Verfahren bereitzustellen, mit dem zuverlässig die Qualität von IgG Antikörpern getestet werden kann, insbesondere eine Schädigung von IgG Antikörpern nachweisbar ist.

Diese Aufgabe wird erfindungsgemäß durch das im Anspruch 1 näher gekennzeichnete Verfahren zum Nachweis einer irreversiblen Schädigung von IgG Antikörpern mit den nachfolgenden Schritten gelöst:
(a) Inkontaktbringen von IgG Antikörpern oder IgG Antikörperpräparationen, die gegen ein Oberflächenantigen auf einer Fcγ-Rezeptor RII und/oder RIII exprimierenden Zellinie gerichtet sind und die im nativen Zustand an den Fcγ-Rezeptor RII oder RIII binden können, mit einer in Suspensionskultur wachsenden, Fcγ Rezeptor RII und/oder RIII und das Oberflächenantigen exprimierenden Zellinie;
(b) Zugabe eines zweiten Antikörpers, der gegen die Ligandenbindungsstelle des Fcγ-Rezeptors RII oder RIII gerichtet ist, zur im Schritt (a) erhaltenen Mischung;
(c) Analyse des Aggregationsverhaltens der Zellen zur Bestimmung einer möglichen Schädigung der IgG Antikörper oder der IgG Antikörperpräparation, wobei
   α) eine Aggregation der Zellen eine Schädigung des IgG Antikörpers oder der IgG Antikörperpräparation anzeigt; und
   β) keine Aggregation oder eine reversible, unspezifische Aggregation die Unversehrtheit des IgG Antikörpers oder der IgG Antikörperpräparation anzeigt.

Weitere bevorzugte Ausgestaltungen der Erfindung ergeben sich aus den Unteransprüchen sowie der nachfolgenden Beschreibung im Zusammenhang mit den Ausführungsbeispielen. Die Ausführungsbeispiele geben eine spezielle Ausgestaltungsform des erfindungsgemäßen Verfahrens wieder, auf das die Erfindung jedoch nicht beschränkt ist.

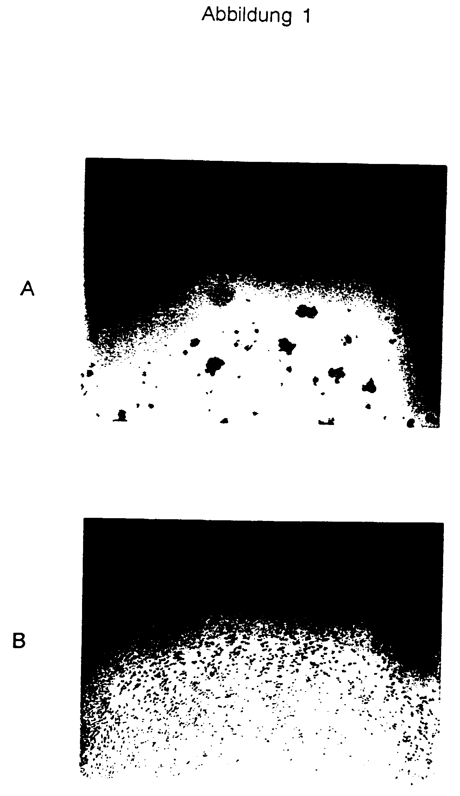

Die beiliegenden Abbildungen 1 A und B zeigen:

S49.1 Zellen (in 50 µl Kulturmedium) wurden in die Kavitäten einer 96 well Mikrotiterplatte pipettiert und mit 100 µl einer MmT1-Verdünnung (A) oder dem gleichen Volumen eines Gemisches aus MmT1 und 2.4G2uk (B) über Nacht inkubiert. Vor der visuellen Auswertung mit Hilfe eines Durchlichtmikroskops wurde die Platte kräftig geschüttelt.

Abbildung 1A zeigt das typische Beispiel einer homotypischen Aggregation von S49.1 Zellen, die durch den anti-Thy-1 Antikörper MmT1 ausgelöst wurde. Im Abbildung 1B ist eindeutig erkennbar, daß S49.1 Zeilen als Einzelzellen den Boden der Mikrotiterplatte bedecken. Im diesem Ansatz wurde die typische MmT1-induzierte Aggregatformation durch die gleichzeitige Anwesenheit des Antikörpers 2.4G2uk im Reaktionsgemisch inhibiert.

Für den Nachweis einer irrversiblen Schädigung von IgG Antikörpern wurde erfindungsgemäß ein in vitro Test etabliert, der von einer in Suspensionskultur wachsenden Zellinie (Indikatorzellen) ausgeht, die zum einen ein Oberflächenantigen exprimiert, gegen welches der IgG Antikörper gerichtet ist und zum anderen die Fcγ-Rezeptoren RII (CD32) und/oder RIII (CD16). Wichtig ist, daß die Anzahl der Fcγ-Rezeptoren im Zusammenspiel mit dem Antigen ausreichend ist, um deutlich nachweisbare Aggregate auszubilden. Hierzu müssen die Antigene in einer bestimmten Kopienanzahl auf der in Suspensionskultur wachsenden Indikatorzellinie exprimiert sein. Es hat sich erfindungsgemäß als günstig herausgestellt, daß das Antigen, gegen welches der IgG Antikörper gerichtet ist, auf der Indikatorzellinie in hoher oder sogar sehr hoher Dichte exprimiert wird, während die Fcγ-Rezeptoren in geringerer Kopienzahl exprimiert sein können.

Das Verhältnis Antigen : Fcγ-Rezeptoren liegt beispielsweise bevorzugt im Bereich von > 1:1 bis 0,5:1. Es sind solche Zellinien auszuwählen, die ein Verhältnis von Antigen zu Fcγ-Rezeptoren aufweisen, daß eine Aggregatbildung möglich ist. Da das Vehältnis von Antigen : Fcγ-Rezeptoren von Zellinie zu Zellinie unterschiedlich ist, ist vor Etablierung des erfindungsgemäßen Verfahrens die Indikatorzellinie auf ihre Fähigkeit zur Aggregatbildung vorab zu testen. Aufgrund der vorliegenden Beschreibung ist ein Austesten für den Fachmann ohne weitere erfinderische Schritte möglich.

Die Zellen werden beispielsweise in einem Standard-Nährmedium in einer Dichte von 10⁵ - 10⁶ Zellen/ml gezogen.

Bei den Indikaktorzellen handelt es sich um tierische Zellen, die das Antigen, gegen den der zu testende IgG Antikörper gerichtet ist, zusammen mit den Fcγ RII und/oder RIII Rezeptoren exprimieren. Die Zellen können entweder natürlicherweise das Antigen und die Fcγ-Rezeptoren exprimieren oder die für das Antigen und/oder die Fcγ-Rezeptoren kodierenden Gene wurden durch rekombinante DNA-Techniken in die Zelle eingeführt. Beispiele für erfindungsgemäß verwendbare Zellen sind alle nichtadhärent wachsenden Zellinien, bevorzugt B-Lymphozyten oder T-Lymphozyten, Granulozyten, natürliche Killerzellen und Mastzellen. Eine besonders bevorzugte Zellinie ist die Zellinie S49.1, die in sehr großer Dichte das Thy-1 Antigen und gleichzeitig, in deutlich geringerer Kopienzahl, den Fcγ-Rezeptor RIII (CD16) exprimiert.

Die Zellinie S49.1 ist bei der Non-profit Organisation American Type Culture Collection (ATCC) unter der Bezeichnung ATCC TIB 28 von Dr. AW Harris (Salk Institute, San Diego, California) hinterlegt worden und kann von dort ohne besondere Auflagen bezogen werden. Referenzen zu dieser Zellinie sind Ralph, P.(1973) und Burgeois, S. et al (1977).

Beim Thy-1 (Theta)-Antigen (CD 90) handelt es sich um ein Oberflächenantigen, welches als Marker für murine T-Zellen fungiert. Referenzen zum Thy-1 Antigen sind beispielsweise Schlesinger, M. et al, 1989, Thierfelder, S. et al, 1989, Cobbold, S.P. et al, 1983, Kummer, U. et al, 1993 und Williams, A.F., 1989.

Es ist nochmals darauf hinzuweisen, daß die Zellinie S49.1 zwar eine erfindungsgemäß bevorzugt einsetzbare Indikatorzellinie ist, die Erfindung jedoch nicht auf diese Zellinie beschränkt ist. Zur Induktion einer homotypischen Zellaggregation kann die Zellinie S49.1 auch durch andere Zellinien ersetzt werden. Allerdings muß gewährleistet sein, daß die ausgewählte Zellinie als Suspensionskultur wächst, d.h. adhärente Zellen sind für die erfindungsgemäß einzusetzende Indikatorzellinie ungeeignet. Weiterhin muß die Zellinie mindestens einen der beiden niedrig-affinen Fcγ-Rezeptoren RII oder RIII exprimieren. Es sind jedoch auch Zellinien einsetzbar, die beide Fcγ-Rezeptoren aufweisen.

Zur Durchführung des Tests bringt man eine Suspension der Indikatorzellen mit einem Antikörper oder einer Antikörperpräparation, die gegen das von der Indikatorzellinie exprimierte Antigen gerichtet ist, zusammen und läßt das Reaktionsgemisch für einen Zeitraum aufeinander einwirken, der ausreichend ist, um eine mögliche Aggregation zu erhalten. Als Antikörper bzw. Antikörperpräparation können monoklonale oder polyklonale Antikörper eingesetzt werden. Entscheidend ist, daß die IgG Antikörper oder IgG Antikörperpräparationen, die im erfindungsgemäßen Verfahren eingesetzt werden, gegen ein Oberflächenantigen gerichtet sind, welches auf der Indikatorzellinie vorkommt. Ein Beispiel hierfür ist das oben genannte Thy-1-Antigen. Beispielsweise können Antikörper-Spezifitäten eingesetzt werden, die maus- und/oder ratte- und/oder humanspezifisch sind. Selbstverständlich können auch andere Antikörper-Spezifitäten eingesetzt werden. Es muß jedoch sichergestellt werden, daß diese Antikörper in Abwesenheit des blockierenden, anti-Fcγ-Rezeptor-Antikörpers deutlich nachweisbare Aggregate ausbilden. Geeignete Antikörper können durch an sich bekannte Testverfahren ermittelt werden. Es ist hierbei darauf zu achten, daß das betreffende Antigen in einer Kopienanzahl auf der Indikatorzellinie exprimiert wird, die eine deutliche Aggregatformation zuläßt. Eine derartige Indikatorzellinie ist beispielsweise die erfindungsgemäß eingesetzte Zellinie S49.1. Weitere mögliche Zellinien können vom Fachmann aufgrund der vorliegenden Beschreibung mit Hilfe einfacher Versuche ermittelt werden.

Wird nun zu einer Probe der Indikatorzellen ein gegen das Oberflächenantigen gerichteter Antikörper zugegeben, lagern sich die mit dem Antikörper beladenen Einzelzellen zu stabilen Aggregaten zusammen. Der Nachweis, daß diese homotypische Aggregation der Indikatorzellen über die spezifische Interaktion des Fc-Teils des gebundenen Antikörpers mit dem zellständigen Fcγ-Rezeptor erfolgt, ergibt sich aus der Beobachtung, daß in Gegenwart eines Antikörpers, der an die Ligandenbindungsstelle des zellständigen Fcγ-Rezeptors bindet, eine Aggregatbildung ausbleibt. Ein derartiger Antikörper, der an die Ligandenbindungsstelle des zellständigen Fcγ-Rezeptors RII oder RIII bindet, blockiert dessen Funktion, d.h. für eine nachfolgende Indikatorreaktion (Antikörper-Fcγ-Rezeptor-Interaktion) steht der Fcγ-Rezeptor RII oder RIII nicht mehr zur Verfügung. Stattdessen beobachtet man, daß in einem solchen Testansatz die Indikatorzellen eindeutig erkennbar als Einzelzellen verbleiben. In der Aggregationsreaktion können natürlich nur solche IgG Antikörper-Subklassen erfaßt werden, die mit dem niedrigaffinen Fcγ-Rezeptor RII oder RIII interagieren.

Die Anwesenheit des zweiten Antikörpers, der an die Ligandenbindungsstelle des zellständigen Fcγ-Rezeptors RII oder RIII bindet und dessen Funktion blockiert, erlaubt die erfindungsgemäß wichtige Unterscheidung zwischen der Bildung von homotypischen Zell-Aggregaten aufgrund einer spezifischen Interaktion des Fc-Teils des gebundenen Antikörpers mit dem zellständigen Fcγ-Rezeptor und einer unspezifischen Antikörperwirkung aufgrund von Wechselwirkungen zwischen den zellgebundenen Antikörpern. Als Folge von Unregelmäßigkeiten bei der Proteinrückfaltung nach einer Säurebehandlung, wie sie beispielsweise während einer Aufreinigung von Antikörpern durchgeführt werden muß, kommt es nämlich zu intermolekularen homophilen Wechselwirkungen und damit zu einer Aggregation der Indikatorzellinien, die eine Schädigung des IgG Antikörpers bzw. der IgG Antikörperpräparation anzeigen. Das unterschiedliche Verhalten von geschädigten und nicht geschädigten Antikörpern bzw. Antikörperpräparationen ist so erklärbar, daß der Rückfaltungsprozess zum nativ gefalteten Protein nach einer Säure-Denaturierung bei beispielsweise pH 2,7 nicht mehr vollständig verläuft und diese irreversible Schädigung des Proteins Auslöser für die Bildung von "unspezifischen" Zellaggregaten als Folge unerwünschter intermolekularer homophiler Wechselwirkungen zwischen den zellgebundenen, auf ihre Unversehrtheit zu untersuchenden IgG Antikörpern oder IgG Antikörperpräparationen ist.

Bei dem erfindungsgemäßen Verfahren ist es nicht zwingend notwendig, daß der tatsächlich aufzureinigende Antikörper, der häufig teuer ist und nur in geringen Mengen zur Verfügung steht, untersucht wird. Stattdessen kann ein Indikator-Antikörper, beispielsweise der in der vorliegenden Erfindung eingesetzte Antikörper MmT1, stellvertretend für den tatsächlich zu reinigenden Antikörper den gesamten Manipulationen eines ausgewählten Reinigungsverfahrens unterzogen werden. Hierbei hat der Indikator-Antikörper die gleiche Subklasse wie der tatsächlich zu reinigende Antikörper.Bevorzugte Subklassen sind Maus 2a und Ratte 2b. Der Indikator-Antikörper, der dem ausgewählten Reinigungsverfahren unterzogen wurde, wird dann im erfindungsgemäßen, rasch und extrem einfach durchzuführenden Aggregationstest, beispielsweise mit den S49.1-Zellen, unterworfen und auf mögliche reinigungsbedingte unspezifische, homophile Wechselwirkungen hin untersucht.

Die Bildung von Aggregaten erfolgt im allgemeinen bereits kurz nach dem Ansetzen des Tests, beispielsweise nach bereits 30 Minuten. Um eine sichere Aussage zu machen, sollte jedoch abgewartet werden, bis sich große, deutlich sichtbare Zellaggregate, zumindest in dem mitgeführten Kontrollansatz, gebildet haben. Dies ist üblicherweise nach 2 bis 3 Stunden der Fall, wobei jedoch auch über Nacht abgewartet werden und die Auswertung am nächsten Tag erfolgen kann.

Um Falschaussagen zu vermeiden, ist die Durchführung eines Kontrollansatzes notwendig, der mit einem mit Sicherheit ungeschädigten Antikörper durchgeführt wird. Da die verwendeten Indikatorzellen sich auch zufällig zusammenlagern können und so Zellaggregate vortäuschen, ist es empfehlenswert, am Ende der Inkubationszeit kräftig zu schütteln, um zufällige Aggregationen wieder aufzulösen. Durch dieses Schütteln können jedoch Aggregationen, die aufgrund einer Schädigung der IgG Antikörper entstanden sind, nicht beseitigt werden.

Nachfolgend wird die Erfindung anhand von Ausführungsbeispielen näher erläutert.

### Beispiele

Im vorliegenden Beispiel wurden S49.1 Zellen als in Suspensionskultur permanent wachsende Indikatorzellinie eingesetzt, die auf ihrer Oberfläche in sehr großer Dichte das Thy-1 Antigen und gleichzeitig, in deutlich geringerer Kopienzahl, den Fcγ-Rezeptor RIII exprimiert.

Es zeigte sich, daß bei Zugabe eines anti-Thy-1 Antikörpers zu einer Probe dieser Zellen eine stabile Aggregatbildung durch Zusammenlagerung der Antikörper-beladenen Einzelzellen erfolgte.

Der Nachweis, daß diese homotypische Aggregation der S49.1 Zellen über die spezifische Interaktion des Fc-Teils des gebundenen Antikörpers mit dem zellständigen Fcγ-Rezeptor erfolgt, ergibt sich aus der Beobachtung, daß in Gegenwart des Antikörpers 2.4G2uk eine Aggregatformation ausbleibt. Der Antikörper 2.4G2uk bindet an die Ligandenbindungsstelle des zellständigen Fcγ-Rezeptors RIII und blockiert dessen Funktion. Für eine nachfolgende Indikatorreaktion (Antikörper- Fcγ-Rezeptor-Interaktion) steht der Fcγ-Rezeptor RIII dann nicht mehr zur Verfügung. Statt dessen beobachtet man, daß in einem solchen Testansatz S49.1 Zellen eindeutig erkennbar als Einzelzellen den Boden der Mikrotiterplatte bedecken. In der Aggregationsreaktion können natürlich nur solche IgG AntikörperSubklassen erfaßt werden, die mit dem niedrig-affinen Fcγ-Rezeptor RIII interagieren.

Neben dieser spezifischen, durch den Antikörper 2.4G2uk inhibierbaren Aggregation der S49.1 Zellen sind grundsätzlich weitere Möglichkeiten der Formation von Zell-Aggregaten durch Antikörper vorstellbar. Auslöser für die Bildung solcher Zell-Zellaggregate könnten z.B. intermolekulare Interaktionen zwischen Zell-gebundenen Antikörpermolekülen sein, die auf die veränderte Eigenschaft von Antikörper/Antikörperpräparationen zurückgehen und z.B. im Verlaufe von Aufreinigungsprozeduren als Folge von Unregelmäßigkeiten bei der Rückfaltung des Proteins akquiriert werden. Für die Richtigkeit dieser Interpretation spricht auch die Tatsache, daß Antikörper-Aggregate als weitere mögliche Auslöser solcher Zell-Aggregate durch vorherige Ultrazentrifugation (100 000xg für 30 min) aus der Probe entfernt wurden. Basierend auf dem erfindungsgemäß etablierten in vitro Test ist es möglich , solche unerwünschten Eigenschaften von Antikörpern/Antikörperpräparationen gezielt zu untersuchen, die beispielsweise bei den nachfolgend ausgeführten Reinigungsverfahren entstehen können.

Das erfindungsgemäße Nachweisverfahren ist von eminent praktischer Bedeutung, da bei der Reinigung von IgG Antikörpern diese häufig sauren Bedingungen ausgesetzt werden, die oft zu Schädigungen führen.

Die erfindungsgemäß zu testenden Antikörper werden von Hybridomen produziert und als Kulturüberstand dieser Hybridome geerntet. Sie können dann z.B. mit dem Standardverfahren der Affinitätschromatographie gereinigt werden. Die Affinitätschromatographie gehört zu den Reinigungsverfahren mit der höchsten Selektivität. Die Probe (Antikörper im Kulturüberstand) wird dabei an die mit Protein A oder alternativ mit Protein G substitutierte chromatographische Matrix gebunden und anschließend mit einem geeigneten Eluenten desorbiert. Protein A- und Protein G-substituierte Matrices werden bereits in Säulen gepackt kommerziell vertrieben.

In den letzten 15 Jahren hat Protein A eine große Bedeutung in der schnellen Ein-Schritt-Affinitätschromatographie zur Reinigung von IgG Antikörpern aus unterschiedlichsten Ressourcen (Aszites, Kulturüberstand) gewonnen. Das bakterielle Polypeptid bindet mit seinem Immunglobulin-bindenden Bereichen mit sehr unterschiedlicher Affinität selektiv an den Fc-Teil aller Säugetier IgG Antikörper. Mit Ausnahme der IgG1 Subklasse verfügen die übrigen Maus IgG Antikörper über eine mittlere Affinität gegenüber Protein A. Aus praktischen Gründen wird meist mit einem einzigen Puffer im Bereich von pH 4,0 eluiert.

Für die Präparation von schlecht Protein A-bindenden IgG Antikörpern (z.B. fast allen Ratten IgG Subklassen) steht neuerdings mit Protein G ein weiterer bakterieller Rezeptor zur Verfügung, der sich besonders für die Aufreinigung von Ratten Immunglobulinen eignet. Wegen der hohen Bindungskonstante zwischen IgG Antikörpern und Protein G erfordert die Ablösung der gebundenen Antikörper relativ saure Bedingungen. Aus praktischen Gründen erfolgt die Elution bei Protein G bei einem pH von 2,7. Gerade diese pH Werte sind jedoch nachteilig.

### Durchführung des Aggregationstests:

Zur Durchführung des Tests bringt man eine Suspension von S49.1 Zellen (typischerweise 5x10⁴ Zellen in 50 µl Kulturmedium) mit eine(m)r Antikörper/Antikörperpräparation (monoklonal oder polyklonal), der/die gegen das Thy-1 Antigen gerichtet ist, in der Kavität einer 96 well Mikrotiterplatte zusammen und läßt das Reaktionsgemisch für mehrere Stunden stehen. Dabei sind weder die Reihenfolge der Zugabe der Reaktanden noch das Volumen des Inkubationssansatzes (zwischen 100 - 200 µl) kritische Größen. Nach dieser Zeit sind in den Ansätzen charakteristische Zell-Aggregate am Boden der Vertiefung der Mikrotiterplatte entstanden, die visuell unter Zuhilfenahme eines Durchlichtmikroskops beurteilt werden (Abbildung 1). Kräftiges Schütteln der Platte am Ende der Inkubationszeit beugt möglichen Schwierigkeiten bei der Auswertung vor, wobei diese darauf beruhen könnten, daß sich S49.1-Zellen zufällig zusammenlagern und so Zell-Aggregate vortäuschen.

Für den Test werden üblicherweise Verdünnungsreihen (1 Teil Antikörper plus 1 Teil Kulturmedium) hergestellt, wobei in der stärkeren Verdünnung eines anti-Thy-1 Antikörpers keine Zell-Aggregate mehr auftreten.

Die Anwesenheit des Antikörpers 2.4G2 uk (im Überschuß) in einem mitgeführten Parallelansatz erlaubt die wichtige Unterscheidung zwischen der Formation von homotypischen Zell-Aggregaten aufgrund von spezifischer (Interaktion des Fc-Teils des gebundenen Antikörpers mit dem zellständigen Fcγ-Rezeptor) oder unspezifischer (Wechselwirkungen zwischen den zellgebundenen Antikörpern) Antikörperwirkung.

Bei diesen Experimenten werden üblicherweise zwei Kontrollansätze mitgeführt. In einem Ansatz befinden sich S49.1 Zellen zusammen mit einem beliebigen Kontroll-Antikörper, der keine Reaktivität gegenüber der S49.1-Zellen aufweist, aber der IgG Subklasse des zu untersuchenden anti-Thy-1 Antikörpers zugehörig ist, während in den zweiten Inkubationsansatz nur der Antikörper 2.4G2uk zugegeben wird.

Im Anschluß wurden vergleichende Versuche durchgeführt, bei denen verschiedene Präparationen eines anti-Thy-1 Antikörpers (MmT1; Maus IgG Subklasse 2a) im S49.1-Aggregationstest untersucht wurden. Es handelt sich dabei um Antikörper-Präparationen, die einem sauren Milieu von pH 2,7 bzw. 4,0 ausgesetzt wurden. Diese Manipulation entspricht jenen Bedingungen, die bei der Ein-Schritt-Affinitätschromatographie mit Protein G (pH 2,7) bzw. A (pH 4,0) verwendet werden, um gebundene maus-Antikörper der Subklasse IgG2a vom Liganden abzulösen.

Bei diesen Versuchen zeigte sich, daß MmT1-Präparationen, die einem pH Wert von 2,7 ausgesetzt waren (entspricht den Verhältnissen bei der Aufreinigung mit Protein G), Zell-Aggregate ausbilden, die durch den Antikörper 2.4G2uk nicht inhibierbar sind. Nachdem die Antikörperpräparation zuvor durch Ultrazentrifugation von Protein-Aggregaten geklärt wurde, erfolgt die Formation von Zell-Aggregaten hier ganz offensichtlich durch unspezifische Wechselwirkungen zwischen den zellgebundenen Antikörpern. Im Gegensatz dazu ist die Formation von Zell-Aggregaten durch MmT1-Präparationen, die einem pH Wert von 4,0 ausgesetzt waren (entspricht den Verhältnissen bei der Aufreinigung mit Protein A), durch den Antikörper 2.4G2uk hemmbar und muß infolgedessen auf spezifischen Interaktionen der Fc-Teile der gebundenen Antikörper mit den zellständigen Fcγ-Rezeptoren beruhen.

Das unterschiedliche Verhalten der beiden Antiköper-Präparationen kann nur so erklärt werden, daß der Rückfaltungssprozess zum nativ gefalteten Protein nach SäureDenaturierung bei pH 2,7 nicht mehr vollständig verläuft. Diese irreversible Schädigung des Proteins ist der Auslöser für die Bildung von "unspezifischen"Zell-Aggregaten als Folge von unerwünschten intermolekularen homophilen Wechselwirkungen zwischen den Zell-gebundenen MmT1 Antikörpern.

Mit Hilfe des S49.1-Aggregationstests konnte dann der überaus wichtige Nachweis erbracht werden, daß nicht nur der Antikörper MmT1 durch Säure-Denaturierung irreversibel geschädigt wird. Vielmehr trifft diese Eigenschaft auch auf andere Antikörper der Maus IgG2a Subklasse zu. In diese Untersuchungen wurden zwei weitere anti-Thy-1 Antikörper der Maus IgG2a Subklasse (MmT5 und MmTC) mit aufgenommen (siehe Tabelle). Aus den Ergebnissen dieser Untersuchungen läßt sich die folgende Regel ableiten: Die Zugehörigkeit eines Antikörpers zu einer bestimmten IgG-Subklasse scheint im Hinblick auf irreversible Veränderungen/Beeinträchtigungen (z.B. durch Säure-Denaturierung) eine bestimmende Größe zu sein.

Genau diese Subklassen-restringierte Eigenschaft ermöglicht es, daß Antikörper wie der MmT1 stellvertretend für andere Antikörper-Spezifitäten den gesamten Manipulationen eines ausgewählten Reinigungsverfahrens unterzogen werden, um sie anschließend in dem rasch und extrem einfach durchzuführenden Aggregationstest auf mögliche Reinigungs-bedingte homophile Wechselwirkungen hin zu untersuchen. Diesem Sachverhalt kommt umsomehr Bedeutung zu, als Maus IgG2a Antikörper (Panorex; OKT3) als sog. protektive Subklasse beim Menschen therapeutisch eingesetzt werden.

Die bei den obenstehend beschriebenen Versuchen eingesetzten Antikörper wurden in der Spezies Maus generiert und gehören der IgG-Klasse und der Subklasse IgG2a an. Diese Antikörper - Subklasse wird wie die Ratten IgG2b-Subklasse den sog. "protektiven Antikörpern" zugerechnet. Diese Bezeichnung ergab sich aus Studien im Tiermodell, in denen gefunden wurde, daß sich beide Antikörper-Subklassen optimal dazu eignen, beispielsweise in vivo Tumorzellen zu eliminieren. In weiteren Versuchen wurden mit dem erfindungsgemäß bereitgestellten Aggregationstest auch Ratten IgG2b Antikörper (3 verschiedene IgG2b Antikörper, ebenfalls mit anti-Thy-1 Spezifität) untersucht; hierbei konnten die bei den Maus IgG2a anti-Thy-1 Antikörpern gewonnenen Ergebnisse bestätigt werden, d.h. auch eine Reinigung eines Ratten IgG2b Antikörpers bei einem stark sauren pH Wert von beispielsweise 2,7 führte im Test zur Aggregation der Zellen und zeigte eine irreversible Schädigung des gereinigten Antikörpers an.

### LITERATURLISTE

1. Burgeois, S., Newby, R.F.: Diploid and haploid states of the glucocorticoid receptor gene of mouse lymphoid cell lines. Cell 11, 423-430 (1977).
2. Cobbold, S.P., Thierfelder, S. and Waldmann, H., Immunosuppression with monoclonal antibodies. A model to determine the rules for effective serotherapy. Mol. Biol. Med. 1983. 1: 285-304.
3. Kummer, U., Haunschild, J., Reisbach, G., Delecluse, H.-J. and Thierfelder, S., Mitogenicity of anti-Thy-1 monoclonal antibodies attributable to an Fc-dependent mechanism. Eur.J Immunol 1993. 23: 2649-2654.
4. Norman, D.J., Kahana, L., Stuart, F.P.J., Thistlewaite, J.R.J.,. Shield, C.F., Manaco, A., Dehlinger, J., Wu, S.C., Van Horn, A., Haverty T.P., 1993. A randomized clinical trial of induction therapy with OKT3 in kidney transplantation. Transplantation 55: 44-50.
5. Ralph, P.: Retention of lymphocyte characteristics by myelomas and θ⁺-lymphomas: sensitivity to cortisol and phytohemagglutinin. J. Immunol., 110, 1470-1475 (1973).
6. Riethmüller, G., Schneider-Gädicke, E., Johnson J.R.. 1993. Monoclonal antibodies in cancer therapy. Curr. Opin. Immunol., 5: 732-739.
7. Riethmüller, G., Schneider-Gädicke, E., Schlimock G. et al. 1994. Randomized trial of monoclonal antibody for adjuvant therapy of resected Dukes C colorectal carcinoma, Lancet, 343: 1177-1183.
8. Schlesinger, M., in Reif, A.E. and Schlesinger, M. (Eds.), Cell Surface Antigen Thy-1. Immunology, Neurology, and Therapeutic Applications, Marcel Dekker, Inc., New York, Basel 1989, S.589.
9. Thierfelder, S., Kummer, U. and Hoffmann-Fezer, G., Anti-Thy-1 in bone marrow transplantation. Immunol. Ser. 1989. 45: 531-572.
10. Williams, A.F., in Reif, A.E. and Schlesinger, M. (Eds.), Cell Surface Antigen Thy-1. Immunology, Neurology, and Therapeutic Applications, Marcel Dekker, Inc., New York, Basel 1989, S.49.

**Tabelle**

| Auflistung der verwendeten monoklonalen Antikörper | | |
|---|---|---|
| Antikörper | Spezifität | IgG Subklasse |
| MmT1 | Thy-1.2 | Maus IgG2a |
| MmT5 | Thy-1.2 | Maus IgG2a |
| MmTc | Thy-1.2 | Maus IgG2a |
| 2.4G2uk | Ligandenbindungsstelle der niedrig-affinen FcγRezeptoren RII und RIII | Ratte IgG2b |

## Patentansprüche

1. Verfahren zum Nachweis einer irreversiblen Schädigung von IgG Antikörpern mit den nachfolgenden Schritten:
(a) Inkontaktbringen von IgG Antikörpern oder IgG Antikörperpräparationen, die gegen ein Oberflächenantigen auf einer Fcγ-Rezeptor RII und/oder RIII exprimierenden Zellinie gerichtet sind und die im nativen Zustand an den Fcγ-Rezeptor RII oder RIII binden können, mit einer in Suspensionskultur wachsenden, Fcγ-Rezeptor RII und/oder RIII und das Oberflächenantigen exprimierenden Zellinie;
(b) Zugabe eines zweiten Antikörpers, der gegen die Ligandenbindungsstelle des Fcγ-Rezeptors RII oder RIII gerichtet ist, zur im Schritt (a) erhaltenen Mischung;
(c) Analyse des Aggregationsverhaltens der Zellen zur Bestimmung einer möglichen Schädigung der IgG Antikörper oder der IgG Antikörperpräparation, wobei
α) eine Aggregation der Zellen eine Schädigung des IgG Antikörpers oder der IgG Antikörperpräparation anzeigt; und
β) keine Aggregation oder eine reversible, unspezifische Aggregation die Unversehrtheit des IgG Antikörpers oder der IgG Antikörperpräparation anzeigt.

2. Verfahren nach Anspruch 1,
dadurch gekennzeichnet, daß
als IgG Antikörper ein Antikörper der Subklasse 2a oder 2b eingesetzt wird.

3. Verfahren nach Anspruch 1 oder 2,
dadurch gekennzeichnet, daß
als IgG Antikörper ein Human-Antikörper oder Nager-Antikörper, beispielsweise Maus- oder Ratten-Antikörper oder ein Inter-Spezies-Antikörper eingesetzt wird.

4. Verfahren nach einem oder mehreren der vorhergehenden Ansprüche,
dadurch gekennzeichnet, daß
der IgG Antikörper gegen ein Oberflächenantigen gerichtet ist, das als Marker für T-Zellen dient.

5. Verfahren nach einem oder mehreren der vorhergehenden Ansprüche,
dadurch gekennzeichnet, daß
als Antikörper ein anti-Thy-1 Antikörper eingesetzt wird.

6. Verfahren nach einem oder mehreren der vorhergehenden Ansprüche,
dadurch gekennzeichnet, daß
als Fcγ-Rezeptor RII und/oder RIII exprimierende Zellen B-Lymphozyten, T-Lymphozyten, Granulozyten, natürliche Killerzellen oder Mastzellen eingesetzt werden.

7. Verfahren nach Anspruch 6,
dadurch gekennzeichnet, daß
als Zellinie S49.1 (ATCC TIB 28) eingesetzt wird.

8. Verfahren nach einem oder mehreren der vorhergehenden Ansprüche,
dadurch gekennzeichnet, daß
der IgG Antikörper ein monoklonaler oder polyklonaler Antikörper ist.
